# EUROPEAN PATENT SPECIFICATION

(11) **EP 2 503 977 B1**
(45) Date of publication and mention of the grant of the patent: **10.06.2015**
(21) Application number: 10790602.6
(22) Date of filing: 11.11.2010
(51) Int. Cl.: A61J 1/03, A61M 15/00, B65D 75/36

(54) **BLISTER STRIP CARRYING DRY POWDER COMPOSITION**
BLISTERSTREIFEN MIT EINER TROCKENPULVERZUSAMMENSETZUNG
PLAQUETTE THERMOFORMÉE PORTANT UNE COMPOSITION DE POUDRE SÈCHE

(30) Priority: 23.11.2009 TR 200908847
(43) Date of publication of application: 03.10.2012
(73) Proprietor: Sima Patent ve Lisanslama Hizmetleri Ltd.Sti., Esenler/Istanbul (TR)
(72) Inventor: BILGIC, Mahmut, Merter / Istanbul (TR)
(86) International application number: PCT/TR2010/000222
(87) International publication number: WO 2011/062568

(56) References cited:
- EP-A1- 0 703 157
- EP-A1- 0 905 042
- DE-A1-102005 035 705

## Description

The present invention relates to a peelable blister strip which is used for carrying dry powder medicament delivered to the patient by inhalation route, according to the preamble of claim 1.

Respiratory system diseases, especially asthma and chronic obstructive pulmonary disease, are among the health problems that people have frequently encountered for many years. It is thought that the delivery of the medicaments which are used locally for the treatment of these diseases cause more effective and rapid results in comparison to the ones that are used systemically. Because local delivery of the medicament causes both a quick response of the target area to the treatment due to the fact that the medicament directly reaches to said target area, and also a considerable decrease in the possibility of occurance of undesirable side effects that are caused by delivery of the medicaments systemically is observed. For this reason, some inhalation devices are designed to be used for the delivery of the medicaments locally.

Said inhalation devices are designed according to the form of the medicament which is delivered to the patient wherein said medicament can be in dry powder form or liquid form. The medicaments in the liquid form are mainly classified into two groups: one group comprises medicaments which include propellant, and other group comprises medicaments which do not include propellant. Therefore, inhalation devices are mainly classified into three groups which are, dry powder inhalers used for delivery of the medicaments in dry powder form, metered dose inhalation devices used for delivery of the medicaments in liquid form with propellant, and nebulization devices used for delivery of the medicaments in liquid form without propellant.

However, the medicaments used for the inhalation therapy is preferably delivered in dry powder form because of the fact that possible drawbacks (stability, easily deformation, uncontrolled dosage vs) that are encountered while using the medicaments in liquid form.

Dry powder inhalation devices comprising blister strip are preferred rather than other types of inhalation devices because the devices comprising blister strip; comprise multiple doses, maintain the stability of the dry powder composition for a long time and has user friendliness provided by the working mechanism. In addition, these devices enable to achieve a hygienic inhalation since the patient never contacts with the blister pack included in said inhalation devices.

In dry powder inhalation devices comprising a blister strip, the blister strip is pierced or delaminated for making each dose of the dry powder medicament that is carried in blister cavity, which are arranged regularly on the blister strip, ready for inhalation.

In every use of the dry powder inhalation device comprising blister strip opened by piercing, one blister of the blister strip is needed to be pierced for preparing one dose of dry powder composition for inhalation. For this reason, at least one needle is included in the device as a piercing means. An unopened blister which is positioned as a result of the advancement of blister strip is pierced by means of at least one needle and hence the dry powder medicament in said blister is ready for inhalation. The dry powder medicament in the opened blister is drawn out of the opened blister through the holes by an airflow which is either an active airflow provided by breath of the patient or a passive airflow provided by pressure pumps or other similar means to be delivered to the patient.

In every use of the dry powder inhalation devices comprising a peelable blister strip, blister strip is delaminating as it is moving forward and hence a blister is opened to inhale one dose of the dry powder medicament contained in said blister. The dry powder medicament in the opened blister is entrained to the patient by an active or passive airflow.

In the prior art, because of the fact that the blister strip does not advance with the same distance in every use of the dry powder inhalation device, some problems, which comprise opening one of the blisters containing one dose of dry powder medicament incompletely or opening more than one blister and cause the uncontrolled dosage of the dry powder medicament, have been encountered. For that reason, insufficient or excessive dose of the dry powder medicament is delivered to patients. Therefore, the delivery of the medicament to the patient in uncontrolled dose prevents accomplishment of an effective treatment due to the delivery of insufficient dose of the medicament or moderate or severe side effects are seen due to the delivery of excessive dose of the medicament.

For that reason, in the prior art, various solutions are found to achieve the advancement of the blister strip with the same distance for holding the opened blister strip in a correct, accurate and safe position for the purpose of providing the delivery of the dry powder medicament in the effective and controlled dose in every use of dry powder inhalation device.

US-A-2008/072898 discloses a blister strip which includes two particular lateral profiles to provide the advancement of said blister strip with the same distance in each actuation of the device. However, since the advancement of the blister strip including this kind of lateral profiles required application of much more force by the patient, the user friendliness of the device containing said blister strip is affected adversely.

WO-A-01/72605 and US 5,497,763 disclose a blister strip having holes in lateral sides and it is aimed to advance blister strip properly in each actuation of the device by the help of at least one gear wheel that engages said holes. However, making holes in the lateral sides of said blister and providing gear wheel to engage the holes easily requires that the width of the blister strip should be larger than the usual. Thus, this case affects the complexity, size and manufacturing cost of the inhalation device containing the blister strip.

The blister strips produced to be used for carrying the dry powder medicament, are structured from a base sheet, at which the blister cavties containing a sufficient amount of dry powder medicament are juxtaposed, and a lid sheet, which acts as a cover for said blister cavities. Said sheets consist of an aluminium foil that is a strong moisture barrier, at least one polymeric layer made from polymeric materials, and preferably other layers, in order to prevent decomposition and agglomeration of the dry powder composition which may arise from the moisture contained in blisters. Because of the fact that the base sheet and the lid sheet of the blister strip consist of a lot of layers and/or the thickness of these layers is too much, the movement of blister strip in the inhalation device becomes difficult. Therefore, the force required for moving the mechanical components used in dry powder inhalation device to advance the blister strip increases and hence the effectiveness of these mechanical components decrease. Besides this case affects the user friendliness of the device adversely, it also affects the accuracy of the position of blister carrying the dry powder medicament to be inhaled.

The fact that the base sheet and the lid sheet of the blister strip consist of a few layers or/and the thickness of these layers is low, may not be sufficient for providing the needed moisture barrier. Since this case may increase the possibility of slipping of the blister strip which is advanced by each actuation of the dry powder inhalation device, it may be difficult to provide the blister strip to be in an accurate position.

In addition to aluminium foil, polymeric layer and/or polymeric layers, various other layers can be used for the formation of the base sheet and the lid sheet of the blister strip. One of said various layers is a paper layer which is used for improving heat and moisture permeability of the blister strip. However, in the manufacture of said blister strip which contains at least one paper layer in its sheets, various drawbacks may be encountered during the stage of cutting the blister strip. Because of the fact that the cutter does not cut the blister strip properly to make said blister strip convenient for use in the dry powder inhalation device, the blister strip may not delaminate completely while it is advanced in the inhalation device. Therefore, the next blister containing the dry powder medicament to be inhaled may not be opened completely and the dry powder medicament is not delivered to the patient sufficiently and effectively.

Because of all these reasons, there is a need to provide a blister strip that is user- friendly, reliable and cost effective for the delivery of dry powder medicament to the patient in an effective and controlled way.

The object of the present invention is to provide a blister strip, which is convenient to be used in the dry powder inhalation device, to deal with above mentioned drawbacks.

Particularly, the object of the present invention is to provide a blister strip which is simple, reliable and cost effective and does not require an inhalation device that is big in size and has a complex mechanism, to meet said requirements for the delivery of the dry powder medicament in an effective and controlled way.

Another object of the present invention is to provide a blister strip which provides placement of the opened blister of said blister strip in a correct and accurate position as a result of advancing easily with each actuation of the device.

Another object of the present invention is to provide a blister strip which is advanced easily, space-saving and which has optimum rigidity and furthermore which causes the mechanical components in inhalation device to work effectively.

The blister strip in accordance with the present invention comprises the features of claim 1. It is structured from the base sheet, at which blisters, each of them contains one dose of the dry powder medicament, are juxtaposed with the same distance, and the lid sheet which closes and seals the blisters. Both of these sheets consist of the layers. These layers forming the sheets of the blister strip in accordance with the present invention comprises an aluminium foil and at least one polymeric layer.

These layers forming both the base sheet and the lid sheet of the blister strip must have a sufficient thickness and properties which provide moisture and gas impermeability to protect the stability of the dry powder medicament which is stored in each blister cavity.

Surprisingly, the inventor have found that the ratio of the volume of the aluminium foil to the volume of the polymeric layers present in a certain range in each sheet of the blister strip which consists of the aluminium foil and at least one polymeric layer, results in that formation of a blister strip which has optimum rigidity; causes that the advancement of the blister strip is achieved easily and hence the components of the inhalation device work effectively and said blister strip delaminates completely. Additionally, a desirable moisture and gas impermeability are provided in said sheets. Because of the fact that there is no need for the inhalation device containing the blister strip in accordance with the present invention to contain additional components for providing the blister strip to be advanced properly, the cost of the manufacture of the inhalation device is reduced. In addition to this, because of the rigidity of the blister strip it can be rolled tightly and as a result it occupies less space in the device. Therefore, the blister strip in accordance with the present invention can be used in the inhalation devices which are small in size and this enables said inhalation devices to be used and carried easily.

The blister strip is provided to have optimum rigidity for the purpose of showing the above mentioned properties as a result of that the ratio of the volume of the used aluminium foil to the volume of the used polymeric layer is in the range of 0.1 to 5.0, preferably in the range of 0.2 to 3.0 in each sheet of said blister strip.

The layer of the base sheet of the blister strip contacting with the dry powder medicament is not aluminium foil. The porous structure of aluminium foil and electrostatic forces cause that a part of the dry powder medicament remains in the inner surface of the cavity uninhaled. Therefore, since this case leads to the inhalation of an inadequate amount of the dry powder medicament, the layer covering the inside of the cavity is the polymeric layer to deal with above mentioned drawbacks.

The polymeric materials used for making the polymeric layers which are present in the base sheet and the lid sheet of the blister strip in accordance with the present invention are preferably selected from a group comprising polyethylene (PE), linear low density polyetylene (LLDPE), low density polyethylene (LDPE), medium density polyethylene (MDPE), high density polyethylene (HDPE), polyprophylene (PP), polypropylene cast film (CPP), monoaxially oriented polypropylene film (MOPP), biaxially oriented polypropylene film (BOPP), polyvinyl chloride (PVC), polyester (PET) (non-oriented polyester, monoaxially oriented polyester, biaxially oriented polyester), polystyrene (PS), polyamide (oriented polyamide (OPA)), polyvinylidene chloride (PVDC), polychlorotrifluoroethylene (PCTFE), peelable plastic film types (peelable polyethylene film, etc), other polymer types containing a halogen.

At most one of the polymeric layers used in the blister strip in accordance with the present invention is made from copolymer(s). Acrylonitrile copolymers, cyclic olefine copolymers (COC) and cyclic olefine polymers (COP) are among the copolymer types that are used in the formation of the polymeric layer. Moreover, the multilayered plastic films formed by superimposing the same or different types of polymeric layers are also used as polymeric layers.

According to the invention desiccant agents are added to the polymeric layer, which is either a single layered or a multi-layered polymeric layer, in order to reduce moisture and gas permeability of the polymeric layers for protection of stability of the dry powder formulation contained in the blisters which are juxtaposed at the base sheet of said blister strip. The desiccant agents are silica gel, zeolite, alumina, baucsite, anhydrous calcium sulfate, activated carbon, clay capable of absorbing water. In the manufacture of the blister strip in accordance with the present invention, the desiccant is added to the polymeric materials preferably selected from the group consisting of polyvinyl chloride (PVC), polyprophylene (PP) and polyethylene (PE) which are used for making the polymeric layers. Since low density polyethylene (LDPE) enables the desiccant to disperse in it homogenously and it also enables the thickness of the blister strip to be in a sufficient amount, the desiccant is preferably added to low density polyethylene (LDPE).

At least one surface of the aluminium foil used in the base sheet and/or the lid sheet of the blister strip in accordance with the present invention can be treated with primers such as epoxy resins and polyurethanes.

The aluminium foil and the polymeric layer, which form the base sheet and the lid sheet of the blister strip, may be bonded to each other by using laminer coatings, laminer adhesives and/or adhesive materials such as adhesive agents. Some lamination methods are used to provide that the layers of the sheets of the blister strip in accordance with the present invention are bonded to each other by using certain adhesive materials. The lamination types such as wet lamination, dry lamination, dispersion lamination, polyethylene lamination, coextrusion lamination can be used.

According to the invention, suitable laminar adhesives such as solvent-based adhesives, solvent free adhesives, water-based acrylic adhesives or polyurethane based adhesives, preferably polyurethane based adhesives, are used in the manufacture of the blister strip. The used adhesives may be hardened by the effect of electromagnetic wave.

Additionally, at least one layer of the sheets of the blister strip in accordance with the present invention can be treated by binders such as aliphatic polyesters.

At least one coating method such as extrusion coating, coextrusion coating, sliding coating, curtain coating may be used in the formation of the layers of the blister strip.

Suitable coatings, that can be selected from a group consisting of acrylates, epoxy resins, melamine resins, uric resins, cellulose nitrate, polyurathanes, polyesters or mixturtes thereof, can be treated with above mentioned coating methods. These coatings can be solvent based systems, water based systems or systems that are containing at least one component. These coatings can be hardened by drying, heating, chemicals or radiation.

According to the present invention, a protective laquer can be used as a polymeric layer in the base sheet and/or the lid sheet of the blister strip. Additionally, at least one layer of the sheets of the blister strip in accordance with the present invention may be coated with a heat seal laquer having high heat resistance to maintain the stability of the dry powder medicament for a long time. This heat seal laquer is preferably peelable heat seal laquer.

The adhesive materials, the coating materials, the binding materials and the heat seal laquer by which the layers of the base sheet and the lid sheet of the blister strip in accordance with the present invention are bonded to each other, are omitted in the calculation of the ratio of the aluminium foil to the volume of the polymeric layer.

Furthermore, some treatments or pretreatments such as brushing, chromate treatment, ionizing, ozone, corona, inflammation or plasma treatment can be applied onto the surface of the aluminium foil in order to provide that the aluminium foil used in the blister strip in accordance with the present invention is bonded to binding material, adhesive material and coating strongly.

According to the present invention, the amount of the coating that is used on the layers of the base sheet and/or the lid sheet of the blister strip is in the range of 2.0 - 20.0 g/m², is preferably in the range of 5.0 -17.0 g/m².

According to the present invention, the amount of primer or adhesive material that can be used between the layers of the base sheet and the lid sheet of the blister strip is in the range of 0 - 10.0 g/m², is preferably in the range of 0 - 5.0 g/m².

According to the invention, the blister strip which consists of blisters which have a cavity volume in the range of 20 to 30 mm³ preferably in the range of 21 to 25 mm³, most preferably in the range of 22-23 mm³. Additionally, each blister cavity having the volume described above is filled up to 25-100 %, preferably up to 70-100 %, most preferably up to 90-100 % of the said volume.

Various known techniques can be employed to join the lid and base sheet and hence to seal the blisters of the pelable blister strip. Such methods comprise adhesive bonding, hot metal bonding, hot metal welding, radio frequency welding, laser welding, ultrasonic welding and hot bar sealing. The lid sheet and base sheet of the pelable blister strip are particularly are sealed by cold form sealing methods. Since these cold form sealing methods are conducted at lower temperatures than conventional heat sealing methods. Such cold form sealing methods are of particular utility where the medicament or medicament formulation for containment within the blister is heat sensitive (e. g. degrades or denatures on heating).

According to the present invention, the blisters which are juxtaposed in the blister strip can be in any suitable shapes. Said blisters can have different or same in shape, structure and amount of volume and contain same or different amount of the dry powder medicament with regard to the method of the treatment.

The distance between blister cavities juxtaposed in the blister strip in accordance with the present invention depends on the mechanism used in the device. The blisters, each of which contains one dose of the dry powder medicament, are opened by using any suitable method. Moreover, since the blister strip in accordance with the present invention is preferably peelable blister strip, in each actuation of the device, the lid sheet is peeled apart from the base sheet to open one of the blisters containing one dose of dry powder medicament while the blister strip is advanced.

Said dry powder medicament, which is stored in the blister cavities of the peelable blister strip is produced with methods known in the prior art. The dry powder medicament contains the active agents with the particle size of less than 20 µm. Lactose is used as an excipient. The excipients with fine and coarser particles which have different particle size range, are preferably used in order to provide effective inhalation of the medicament formulation.

Active agent or active agents in dry powder form that are stored in the blister strip present in the dry powder inhaler of the present invention can be selected from a group comprising cromolyns, anti-infectives, antihistamines, anti-inflammatories, bronchodilators, leukotriene inhibitors, PDE IV inhibitors, antitussives, diuretics, anticholinergics, hormones, xanthines. These active agents can be in the form of their salts solvates or esters.

Anticholinergics that are used as an active agent can be selected from a group comprising : tiotropium or tiotropium bromide, oxitropium bromide, flutropyum bromide, ipratropium bromide, glicopyronium salts, trospium chloride, tolterodine, 2,2-diphenylpropionic acid, trophenol ester methobromide, 2,2-diphenylpropionic acid scopine ester metobromide, 2-fluoro-2,2-diphenyl acetic acid scopine ester metobromide, 2-fluoro-2,2-diphenyl acetic acid tropenol ester metobromide, 3,3',4,4'-tetrafluorobenzylic acid tropenol ester metobromide, 3,3',4,4'-tetrafluorobenzylic acid scopine ester metobromide, 4,4'-difluorobenzylic acid tropenol ester metobromide, 4,4'-difluorobenzylic acid scopine ester metobromide, 3,3'-difluorobenzylic acid tropenol ester metobromide, 3,3'-difluorobenzylic acid scopine ester metobromide, 9-hydroxy-fluoren-9-carboxylic acid tropenol ester metobromide, 9-fluoro-fluoren-9-carboxylic acid tropenol ester metobromide, 9-hydroxy-fluoren-9-carboxylic acid scopine ester metobromide, 9-fluoro-fluoren-9-carboxylic acid scopine ester metobromide, 9-methyl-fluoren-9-carboxylic acid tropenol ester metobromide, 9-methyl-fluoren-9-carboxylic acid scopine ester metobromide, benzylic acid cyclopropyltropin ester metobromide, 2,2-diphenylpropionic acid cyclopropyltropine ester metobromide, 9-hydroxy-xanthine-9-carboxylic acid cyclopropyltropin ester metobromide, 9-methyl-fluorene-9-carboxylic acid cyclopropyltropine ester metobromide, 9-methyl-xanthine-9-carboxylic acid cyclopropyltropine ester metobromide, 9-hydroxy-fluorine-9-carboxylic acid cyclopropyltropin ester metobromide, 4,4'-difluorobenzylic acid methylester cyclopropyltropin ester metobromide, 9-hydroxy-xanthine-9-carboxylic acid tropenol ester metobromide, 9-hydroxy-xanthin-9-carboxylic acid scopine ester metobromide, 9-methyl-xanthine-9-carboxylic acid tropenol ester metobromide, 9-methyl-xanthin-9-carboxylic acid scopine ester metobromide, 9-ethyl-xanthine-9-carboxylic acid tropenol ester metobromide, 9-difluoromethyl-xantine-9-carboxylic acid tropenol ester metobromide and 9-hydroxymethyl-xanthine-9-carboxylic acid scopine ester metobromide; preferably their racemates, enantiomers or diastereomers, and solvates and/or hydrates.

Anti-inflammatory agents that are used as active agent are selected from a group comprising prednisolone, prednison, buticocortpropionate, RPR-106541, flunisolid, beklomethasone, triamcinolon, budesonid, flutikazon, mometazon, ciclesonid, rofleponid, ST-126, dexametason, 6α, 9α -difluoro-17α-[(2-furanylcarbonyl)oxy]-11β-hydroxy-16α-methyl-3-oxo-androsta-1,4-diene-17β-carbothionic acid (S)-fluoromethylester, 6α,9α-difluoro-11β-hydroxy-16a-methyl-3-oxo-17α-propionyloxy-androsta-1,4-dien-17β-carbothionic acid (S)-(2-oxo-tetrahydro-furan-3S-yl)ester and ethyprednol-dichloroacetate (BNP-166); preferably racemates, enantiomera or diastereomers, and preferaby their acid addition salts, solvates and/or hydrates.

Leukotriene inhibitors that are used as active agent are selected froma group comprising montelukast, 1-(((R)-(3-(2-(6,7-difluoro-2-quinoil)ethenyl)phenyl)-3-(2-(2-hydroxy-2-propyl)phenyl)thio) mehylcyclopropyl acetic acid, 1-(((1(R)-3(3-(2-(2,3-dichlorothieno[3,2-b]pyridine-5-yl)-(E)-ethenyl)phenyl)-3-(2-(1-hydroxy-1-methylethyl)phenyl)propyl)thio)methyl)cyclopropane acetic acid, pranlukast, zafirlukast, [2-[[2-(4-tert-butyl-2-thiazolyl)-5-benzofuranyl]oxymethyl]phenyl]acetic acid, MCC-847 (ZD-3523), MN-001, MEN-91507 (LM-1507), VUF-5078, VUF-K-8707 and L-733321; preferably racemates, enantiomers or diastereomers, and preferably acid addition salts, solvates and/or hydrates.

PDE IV inhibitor that are used as active agent are selected from enprophilin, teophilin, roflumilast, ariflo (silomilast), CP-325,366, BY343, D-4396 (Sch-351591), AWD-12-281 (GW-842470), N-(3,5-dichloro-1-oxo-pyridin-4-yl)-4-difluoromethoxy-3-cyclopropylmethoxybenzamide, NCS-613, pumaphentine, (-)p-[(4aR*,10bS*)-9-ethoxy-1,2,3,4,4a,10b-hexahydro-8-methoxy-2-methylbenzo[s][1,6]naphthydridine-6-yl]-N,N-diisopropilbenzamide, (R)-(+)-1-(4-bromobenzyl)-4-[(3-cyclopentyloxy)-4-methoxyphenyl]-2-pyrolidone, 3-(cyclopentyloxy-4-methoxyphenyl)-1-(4-N'-[N-2-cyano-S-methyl-isothioureido]benzyl)-2-pyrolidone,cis[4-cyano-4-(3-cyclopentyloxy-4-methoxyphenyl)cyclohexane-1-carboxylic acid], 2-carbomethoxy-4-cyano-4-(3-cyclopropylmethoxy-4-difluoromethoxyphenyl)cyclohexane-1-on, cis[4-cyano-4-(3-cyclopropylmethoxy-4-difluoromethoxyphenyl)cyclohexane-1-ol],(R)-(+)-ethyl[4-(3-cyclopentyloxy-4-methoxyphenyl)pyrolidine-2-ylidine]acetate,(S)-(-)-ethyl[4-(3-cyclopentyloxy-4-methoxyphenyl)pyrolidine-2-ylidin]acetate, CDP840, Bay-198004, D-4418, PD-168787, T-440, T-2585, arophilin, atizoram, V-11294A, C1-1018, CDC-801, CDC-3052, D-22888, YM-58997, Z-15370, 9-cyclopentyl-5,6-dihydro-7-ethyl-3-(2-thienyl)-9H-pyrozolo[3,4-c]-1,2,4-triazolo[4,3-a]pyridine and 9-cyclopentyl-5,6-dihydro-7-ethyl-3-(tert-butyl)-9H-pyrazolo[3,4-c]-1,2,4-triazolo[4,3-a]pyridin; preferably its racemates, enantiomers or diastereomers, and preferably their acid addition salts, solvates and/or hydrates.

Brochodilators used as active agents are preferably selected from salmeterol, salbutamol, formoterol, terbutaline, 3-(4-{[6-({(2fi)∼2-hydroxy-2-[4-hydroxy-3-(hydroxymethyl) phenyl]ethyl}amino)hexyl] oxy} butyl) benzenesulfonamide, 3-(3-{[7-({(2R)-2-hydroxy-2-[4-hydroxy-3-hydroxymethyl)phenyl] ethyl-amino) heptyl] oxy} propyl) benzenesulfonamide, 4-{(1fi)-2-[(6-{2-[(2, 6-dichlorobenzyl)oxy]ethoxy}hexyl) amino]-1-hydroxyethyl} -2-(hydroxymethyl)phenol, N-[2-hydroxyl-5-[(1R)-1-hydroxy-2-[[2-4-[[(2R)-2-hydroxy-2-phenylethyl]amino]phenyl]ethyl]amino]ethyl]phenyl]formamide and N-2{2-[4-(3-phenyl-4-methoxyphenyl)aminophenyl]ethyl}-2-hydroxy-2-(8-hydroxy-2(1H)-quilinolone-5-yl) ethylamine, 5-[(R)-2-(2-{4-[4-(2-amino-2-methyl-propoxy)-phenylamino]-phenyl}-ethylamino)-1-hydroxy-ethyl]-8-hydroxy-1H-quinoline-2-on, preferably their racemates, enantiomers or diastereomers or their acid addition salts, solvates and/or hydrates.

Antihistamines that are used as active agents are preferably selected from the group comprising amelexanose, astemizole azatidine, azelastine, acrivastin, brompheniramine, cetirizine, levocetirizine, efletirizine, chlorpheniramine, clemastine, cyclizin, carebastin, ciproheptadie, carbonoxamine, descarboathoxyloratadine, doxylamine, dimethidene, ebastine, epinastin, efletirizine, fexofenadine, hydroxyzine, ketotifene, loratadin, levocabastine, mizolastine, mequitazine, mianserine, noberastine, meclizine, noracetamizole, olopatadine, pikumast, pyrilamine, prometazine, terfenadine, triphenylamine, temelastine, trimeprazine, triprolidine preferably cetirizine, levocetirizine, efletirizine and fexofenadine and pharmaceutcally acceptable salts, solvates, hydrates thereof.

## Claims

1. A blister strip carrying a dry powder medicament is structured from a base sheet and a lid sheet, wherein the ratio of the volume of the aluminium foil to the volume of the polymeric layer is in the range of 0.1 to 5.0 in each sheet of said blister strip and said polymeric layers used in the base sheet and/or the lid sheet of said blister are single layered- or multilayered-polymeric layers **characterized in that** desiccant agents are added to said single or multilayered polymeric layers and dessicant agent is selected from a group comprising silica gel, zeolite, alumina, bauxite, anhydrous calcium sulfate, activated carbon, clay capable of absorbing water.

2. A blister strip according to claim 1, wherein the ratio of the volume of the aluminium foil to the volume of the polymeric layer is in the range of 0.2 - 3.0 in each sheet of said blister strip.

3. A blister strip according to claim 1, wherein the polymeric materials used for making the polymeric layers which are present in both the base sheet and the lid sheet of the blister are selected from a group comprising polyethylene (PE), linear low density polyetylene (LLDPE), low density polyethylene (LDPE), medium density polyethylene (MDPE), high density polyethylene (HDPE), polyprophylene (PP), polypropylene cast film (CPP), monoaxially oriented polypropylene film (MOPP), biaxially oriented polypropylene film (BOPP), polyvinyl chloride (PVC), polyester (PET) (non-oriented polyester, monoaxially oriented polyester, biaxially oriented polyester), polystyrene (PS), polyamide (oriented polyamide (OPA)), polyvinylidene chloride (PVDC), polychlorotrifluoroethylene (PCTFE), peelable plastic film types (peelable polyethylene film, etc.), other polymer types containing a halogen.

4. A blister strip according to claim 1, wherein at most one of the polymeric layers used in the base sheet and/or the lid sheet of said blister strip is made from copolymers)

5. A blister strip according to claim 1, wherein primer and/or laminer coatings and/or laminer adhesives and/or adhesive agents and/or binding materials are used to provide that said aluminium foil and said polymeric layer, which form the base sheet and the lid sheet of the blister strip, are bonded to each other.

6. A blister strip according to claim 1, wherein the amount of the coating that is used on the layers of the base sheet and/or the lid sheet of the blister strip is in the range of 2.0 - 20.0 g/m².

7. A blister strip according to claim 6, wherein the amount of the coating that is used on the layers of the base sheet and/or the lid sheet of the blister strip is in the range of 5.0 -17.0 g/m².

8. A blister strip according to claim 1, wherein the amount of the primer or the adhesive material that is used between the layers of the base sheet and/or the lid sheet of the blister strip is in the range of 0 - 10.0 g/m².

9. A blister strip according to claim 1, wherein at least one sheet of said blister strip comprising from the outside to the inside of said sheet:
• Protective lac in the range of 1.0 - 3.0 g/m²,
• Aluminium foil in the range of 15.0 - 25.0 µm,
• Primer in the range of 0.5 - 1.5 g/m², and
• Extrusion coating in the range of 10.0 - 20.0 g/m²

10. A blister strip according to claim 1, wherein at least one sheet of said blister strip comprising from the outside to the inside of said sheet:
• Polyester film in the range of 18.0 - 27.0 µm,
• Adhesive material in the range of 1.0 - 5.0 g/m²,
• Aluminium foil in the range of 34.0 - 42.0 µm, and
• Heat seal laquer in the range of 5.0 - 10.0 g/m²

11. A blister strip according to claim 1, wherein at least one sheet of said blister strip comprising from the outside to the inside of said sheet:
• Polyester film in the range of 19.0 - 26.0 µm,
• Adhesive material in the range of 1.0 - 5.0 g/m²,
• Aluminium foil in the range of 22.0 - 28.0 µm,
• Adhesive material in the range of 1.0 - 5.0 g/m², and
• Single layered- or multilayered- peel polymeric layer in the range of 27.0 - 33.0 µm.

12. A blister strip according to claim 1, wherein at least one sheet of said blister strip comprising from the outside to the inside of said sheet:
• Oriented polyamide film in the range of 22.0 - 28.0 µm
• Adhesive agent in the range of 1.0 - 5.0 g/m²
• Primer in the range of 1.0 - 3.0 g/m²
• Aluminium foil in the range of 57.0 - 63.0 µm
• Adhesive agent in the range of 1.0 - 5.0 g/m², and
• Peel polyethylene film in the range of 37.0 - 43.0 µm.

13. A blister strip according to claim 1, wherein at least one sheet of said blister strip comprising from the outside to the inside of said sheet:
• Oriented polyamide film in the range of 22.0 - 28.0 µm
• Adhesive agent in the range of 1.0 - 5.0 g/m²
• Primer in the range of 1.0 - 3.0 g/m²
• Aluminium foil in the range of 57.0 - 63.0 µm
• Adhesive agent in the range of 1.0 - 5.0 g/m², and
• Polypropylene film in the range of 57.0 - 63.0 µm.

14. A blister strip according to claim 1, wherein at least one sheet of said blister strip comprising from the outside to the inside of said sheet:
• Oriented polyamide in the range of 22.0 - 28.0 µm
• Adhesive agent in the range of 1.0 - 5.0 g/m²
• Primer in the range of 1.0 - 3.0 g/m2
• Aluminium foil in the range of 42.0 - 48.0 µm
• Coating in the range of 5.0 - 15.0g/m2, and
• Polyethylene film + desiccant + polyethylene film with coextrusion coating in the range of 38.0 - 43.0 µm

## Patentansprüche

1. Ein Blisterstreifen mit einem Trockenpulver-Medikament wird aus einer Basisfolie und einer Deckfolie aufgebaut, wobei das Verhältnis des Volumens der Aluminiumfolie zum Volumen der polymeren Schicht im Bereich von 0,1 bis 5,0 in jeder Folie des genannten Blisterstreifens ist und die genannten Polymerschichten, die in der Basisfolie und/oder in der Deckfolie des genannten Blisters verwendet werden, ein- oder mehrschichtige Polymerschichten sind, **dadurch gekennzeichnet, dass** Trockenmittel den genannten ein- oder mehrschichtigen Polymerschichten hinzugefügt werden und das Trockenmittel aus einer Gruppe ausgewählt wird, die Silikagel, Zeolith, Aluminiumoxid, Bauxit, wasserfreies Calciumsulfat, Aktivkohle, Lehm, der Wasser absorbieren kann, enthält.

2. Ein Blisterstreifen nach Anspruch 1, wobei das Verhältnis des Volumens der Aluminiumfolie zum Volumen der polymeren Schicht im Bereich von 0,2 bis 3,0 in jeder Folie des genannten Blisterstreifens ist.

3. Ein Blisterstreifen nach Anspruch 1, wobei Polymermaterialien, die zur Herstellung der polymeren Schichten benutzt werden, die sowohl in der Basisfolie als auch in der Deckfolie des Blisters vorhanden sind, aus einer Gruppe ausgewählt werden, die Polyethylen (PE), lineares Polyethylen niedriger Dichte (LLDPE), Polyethylen niedriger Dichte (LDPE), Polyethylen mittlerer Dichte (MDPE), Polyethylen hoher Dichte (HDPE), Polypropylen (PP), Flachtfilmfolie aus Polypropylen (CPP), monoaxial orientierte Polypropylenfolie (MOPP), biaxial orientierte Polypropylenfolie (BOPP) Polyvinylchlorid (PVC), Polyester (PET) (nicht orientierter Polyester, monoaxial orientierter Polyester, biaxial orientierter Polyester), Polystyrol (PS), Polyamid (orientiertes Polyamid (OPA)), Polyvinylidenchlorid (PVDC), Polychlortrifluorethylen (PCTFE) abziehbare Typen der Kunststoffolie (abziehbare Polyethylenfolie, etc.), andere Polymertypen, die ein Halogen enthalten, umfasst.

4. Ein Blisterstreifen nach Anspruch 1, wobei höchstens eine der Polymerschichten, die in der Basisfolie und/oder in der Deckfolie des genannten Blisterstreifens verwendet wird, aus Copolymeren hergestellt wird.

5. Ein Blisterstreifen nach Anspruch 1, wobei Grund- und/oder laminare Beschichtungen und/oder laminare Klebstoffe und/oder Klebemittel und/oder Bindematerialien verwendet werden, um es zu ermöglichen, dass die genannte Aluminiumfolie und die genannte Polymerschicht miteinander verbunden sind, die Basisfolie und Deckfolie des Blisterstreifens bilden.

6. Ein Blisterstreifen nach Anspruch 1, wobei die Menge der Beschichtung, die auf den Schichten der Basisfolie und/oder der Deckfolie des Blisterstreifens verwendet wird, im Bereich von 2,0 bis 20,0 g/m² ist.

7. Ein Blisterstreifen nach Anspruch 6, wobei die Menge der Beschichtung, die auf den Schichten der Basisfolie und/oder der Deckfolie des Blisterstreifens verwendet wird, im Bereich von 5,0 bis 17,0 g/m² ist.

8. Ein Blisterstreifen nach Anspruch 1, wobei die Menge des Grund- oder Klebematerials, das zwischen den Schichten der Basisfolie und/oder der Deckfolie des Blisterstreifens verwendet wird, im Bereich von 0 bis 10,0 g/m² ist.

9. Ein Blisterstreifen nach Anspruch 1, wobei mindestens eine Folie des genannten Blisterstreifens, der von außen nach innen der genannten Platte umfasst:
• Schutzlack im Bereich von 1,0 bis 3,0 g/m²,
• Aluminiumfolie im Bereich von 15,0 bis 25,0 µm,
• Grundierung im Bereich von 0,5 bis 1,5 g/m², und
• Extrusionsbeschichtung im Bereich von 10,0 bis 20,0 g/m².

10. Ein Blisterstreifen nach Anspruch 1, wobei mindestens eine Folie des genannten Blisterstreifens, der von außen nach innen der genannten Folie umfasst:
• Polyesterfolie im Bereich von 18,0 bis 27,0 µm,
• Klebematerial im Bereich von 1,0 bis 5,0 g/m²,
• Aluminiumfolie im Bereich von 34,0 bis 42,0 µm, und
• Heißsiegellack im Bereich von 5,0 bis 10,0 g/m².

11. Ein Blisterstreifen nach Anspruch 1, wobei mindestens eine Folie des genannten Blisterstreifens, der von außen nach innen der genannten Folie umfasst:
• Polyesterfolie im Bereich von 19,0 bis 26,0 µm,
• Klebematerial im Bereich von 1,0 bis 5,0 g/m² ,
• Aluminiumfolie im Bereich von 22,0 bis 28,0 µm,
• Klebematerial im Bereich von 1,0 bis 5,0 g/m², und
• Einschichtige oder mehrschichtige Polymerschicht mit der Schale im Bereich von 27,0 bis 33,0 µm.

12. Ein Blisterstreifen nach Anspruch 1, wobei mindestens eine Folie des genannten Blisterstreifens von außen nach innen der genannten Folie umfasst:
• Orientierte Polyamidfolie im Bereich von 22,0 bis 28,0 µm,
• Klebemittel im Bereich von 1,0 bis 5,0 g/m²,
• Grundierung im Bereich von 1,0 bis 3,0 g/m²,
• Aluminiumfolie im Bereich von 57,0 bis 63,0 µm,
• Klebemittel im Bereich von 1,0 bis 5,0 g/m², und
• Polyethylenfolie mit der Schale im Bereich von 37,0 bis 43,0 µm.

13. Ein Blisterstreifen nach Anspruch 1, wobei mindestens eine Folie des genannten Blisterstreifens, der von außen nach innen der genannten Folie umfasst:
• Orientierte Polyamidfolie im Bereich von 22,0 bis 28,0 µm,
• Klebemittel im Bereich von 1,0 bis 5,0 g/m²,
• Grundierung im Bereich von 1,0 bis 3,0 g/m²,
• Aluminiumfolie im Bereich von 57,0 bis 63,0 µm,
• Klebemittel im Bereich von 1,0 bis 5,0 g/m², und
• Polypropylenfolie im Bereich von 57,0 bis 63,0 µm.

14. Ein Blisterstreifen nach Anspruch 1, wobei mindestens eine Folie des genannten Blisterstreifens, der von außen nach innen der genannten Folie umfasst:
• orientiertes Polyamid im Bereich von 22,0 bis 28,0 µm,
• Klebemittel im Bereich von 1,0 bis 5,0 g/m²,
• Grundierung im Bereich von 1,0 bis 3,0 g/m²,
• Aluminiumfolie im Bereich von 42,0 bis 48,0 µm,
• Beschichtung im Bereich von 5,0 bis 15,0 g/m², und
• Polyethylenfolie + Trockenmittel + Polyethylenfolie mit der Coextrusionsbeschichtung im Bereich von 38,0 bis 43,0 µm.

## Revendications

1. Une bande de blister portant une médicament à poudre sèche est structuré d'une plaque de base et une plaque de couvercle, dans laquelle le rapport entre le volume de la feuille d'aluminium et le volume de la couche polymérique est dans la plage de 0.1 à 5.0 dans chaque plaque de ladite bande de blister et lesdites couches polymériques utilisées dans la plaque de base et/ou la plaque de couvercle dudit blister sont des couches polymériques à monocouche ou multicouches, **caractérisée en ce que**, les agents déshydratants sont ajoutés aux dites couches polymériques à monocouche ou multicouches et l'agent déshydratant est choisi dans la groupe comprenant le gel de silice, la zéolithe, l'alumine, la bauxite, le sulfate de calcium anhydre, le charbon actif, l'argile capable d'absorber l'eau.

2. Une bande de blister selon la revendication 1, dans laquelle, le rapport entre le volume de la feuille d'aluminium et le volume de la couche polymérique est dans la plage de 0.2 - 3.0 dans chaque plaque de ladite bande de blister.

3. Une bande de blister selon la revendication 1, dans laquelle, les matériaux polymériques utilisées pour réaliser les couches polymériques qui sont présents à la fois dans la couche de base et dans la couche de couvercle dudit blister sont choisis dans la groupe comprenant le polyéthylène (PE), le polyéthylène à basse densité linéaire (LLPDE), le polyéthylène à basse densité (LDPE), le polyéthylène à moyenne densité (MDPE), le polyéthylène à haute densité (HDPE), le polypropylène (PP), le film de polypropylène coulé (CPP), le film de polypropylène orienté monoaxialement (MOPP), le film de polypropylène orienté biaxialement (BOPP), le chlorure de polyvinyle (PVC), le polyester (PET) (le polyester non-orienté, le polyester orienté monoaxialement, le polyester orienté biaxialement), le polystyrène (PS), le polyamide (le polyamide orienté(OPA)), le chlorure de polyvinylidène (PVDC), le polychlorotrifluoroethylène (PCTFE), les types des films plastiques épluchables (le film polyéthylène épluchable, etc.), les autres types de polymères contenant un halogène.

4. Une bande de blister selon la revendication 1, dans laquelle, au maximum l'une des couches polymériques utilisée dans la plaque de base et/ou la plaque de couvercle de ladite bande de blister, est réalisé à partir des copolymère(s).

5. Une bande de blister selon la revendication 1, dans laquelle, l'apprêt et/ou les couchages laminaires et/ou les adhésives laminaires et/ou les agents adhésifs et/ou les matériaux liants sont utilisés afin de permettre ladite feuille d'aluminium et ladite couche polymérique qui forment la plaque de base et la plaque de couvercle de la bande de blister à être liés l'une à l'autre.

6. Une bande de blister selon la revendication 1, dans laquelle, la quantité de couchage qui est utilisé sur la plaque de base et/ou la plaque de couvercle de la bande de blister est dans la plage de 2.0 - 20.0 g/m².

7. Une bande de blister selon la revendication 6 dans laquelle, la quantité de couchage qui est utilisé sur la plaque de base et/ou la plaque de couvercle de la bande de blister est dans la plage de 5.0-17.0 g/m².

8. Une bande de blister selon la revendication 1, dans laquelle, la quantité de matériau adhésif qui est utilisé entre la plaque de base et/ou la plaque de couvercle de la bande de blister est dans la plage de 0 - 10.0 g/m².

9. Une bande de blister selon la revendication 1, dans laquelle, au moins l'une des plaques de ladite bande de blister comprenant de l'extérieur vers l'intérieur de ladite plaque:
• Laque protectrice dans la plage de 1.0 - 3.0 g/m²,
• Feuille d'aluminium dans la plage de 15.0 - 25.0 µm,
• Apprêt dans la plage de 0.5 - 1.5 g/m², et
• Couchage par extrusion dans la plage de 10.0 - 20.0 g/m².

10. Une bande de blister selon la revendication 1, dans laquelle, au moins l'une des plaques de ladite bande de blister comprenant de l'extérieur vers l'intérieur de ladite plaque:
• Film polyester dans la plage de 18.0 - 27.0 µm,
• Matériau adhésif dans la plage de 1.0 - 5.0 g/m²,
• Feuille d'aluminium dans la plage de 34.0 - 42.0 µm, et
• Laque de thermoscellage dans la plage de 5.0 - 10.0 g/m².

11. Une bande de blister selon la revendication 1, dans laquelle, au moins l'une des plaques de ladite bande de blister comprenant de l'extérieur vers l'intérieur de ladite plaque:
• Film polyester dans la plage de 19.0 - 26.0 µm,
• Matériau adhésif dans la plage de 1.0 - 5.0 g/m²,
• Feuille d'aluminium dans la plage de 22.0 - 28.0 µm,
• Matériau adhésif dans la plage de 1.0 - 5.0 g/m², et
• Couche d'écorce polymérique à monocouche ou multicouches dans la plage de 27.0-33.0 µm. 12.

12. Une bande de blister selon la revendication 1, dans laquelle, au moins l'une des plaques de ladite bande de blister comprenant de l'extérieur vers l'intérieur de ladite plaque:
• Film polyamide orienté dans la plage de 22.0 - 28.0 µm,
• Agent adhésif dans la plage de 1.0 - 5.0 g/m²,
• Apprêt dans la plage de 1.0 - 3.0 g/m2,
• Feuille d'aluminium dans la plage de 57.0 - 63.0 µm,
• Agent adhésif dans la plage de 1.0 - 5.0 g/m², et
• Film d'écorce polyéthylène dans la plage de 37.0 - 43.0 µm.

13. Une bande de blister selon la revendication 1, dans laquelle, au moins l'une des plaques de ladite bande de blister comprenant de l'extérieur vers l'intérieur de ladite plaque:
• Film polyamide orienté dans la plage de 22.0 - 28.0 µm,
• Agent adhésif dans la plage de 1.0 - 5.0 g/m²,
• Apprêt dans la plage de 1.0 - 3.0 g/m2,
• Feuille d'aluminium dans la plage de 57.0 - 63.0 µm,
• Agent adhésif dans la plage de 1.0 - 5.0 g/m², et
• Film polypropylène dans la plage de 57.0 - 63.0 µm.

14. Une bande de blister selon la revendication 1, dans laquelle, au moins l'une des plaques de ladite bande de blister comprenant de l'extérieur vers l'intérieur de ladite plaque:
• Polyamide orienté dans la plage de 22.0 - 28.0 µm,
• Agent adhésif dans la plage de 1.0 - 5.0 g/m²,
• Apprêt dans la plage de 1.0 - 3.0 g/m2,
• Feuille d'aluminium dans la plage de 42.0 - 48.0 µm,
• Couchage dans la plage de 5.0 - 15.0g/m2, et
• Film polyéthylène + déshydratants + film polyéthylène avec couchage par coextrusion dans la plage de 38.0-43.0 µm.
